# EUROPEAN PATENT SPECIFICATION

(11) **EP 0 733 351 B1**
(45) Date of publication and mention of the grant of the patent: **28.11.2001**
(21) Application number: 96102989.9
(22) Date of filing: 20.09.1991
(51) Int. Cl.: A61F 13/00, A61F 13/06, A61F 13/10, A61F 13/14

(54) **Wearing article with strapping function**
Kleidungsstücke mit Stützwirkung
Article vestimentaire avec fonction de bande de contention

(30) Priority: 22.05.1991 JP 14787391
(43) Date of publication of application: 25.09.1996
(62) Divisional of application: 91308596.5
(73) Proprietor: WACOAL CORPORATION, Kyoto-shi, Kyoto (JP)
(72) Inventor: Fujimoto, Masami, Shimogyo-ku, Kyoto-shi (JP)
(74) Representative: Nettleton, John Victor

(56) References cited:
- GB-A- 2 242 818
- US-A- 4 216 547
- US-A- 4 910 802

## Description

### BACKGROUND OF THE INVENTION

This invention relates to wearing articles or clothes with a strapping function which are adapted to be worn on and tightly fitted on various parts of the human body so as to reinforce the relevant articulations (joints) and muscles.

When having various kinds of sport activities and trainings, or taking various kinds of exercise for fitness purposes, people put on the type of wear which prevents an external wound and prevents the relapse of a wound. In addition, in order to effectively prevent an injury and to provide an effective remedy, there has been widely used a so-called strapping treatment in which a suitable length of tape of an elastic nature or a nonelastic nature is tightly wound on or adhesively bonded tightly to the articulation, the muscle or the ligament of the human body in a perpendicular direction relative to the human body. Namely, the strapping treatment is used to prevent an injury, a wound resulting from fatigue, the relapse of such wound, or to provide an emergency treatment at the site immediately after an accident. After the injury is cured, the strapping is used for reinforcing the cured portion. Originally, the strapping is intended to reinforce the articulation and the muscle, using a medical or an athletic adhesive tape having an acrylic resin adhesive coated on one surface of a substrate of cotton or polyester, and this tape is adapted to be tightly wound on or adhesively bonded to a required portion of the human body so as to prevent an injury, to provide an emergency treatment, to promote rehabilitation and to prevent the relapse of an injury.

Besides such strapping, there have been widely used supporters which are made of an elastic material and adapted to be put on a portion of the human body, such as an ankle and a knee.

However, when the strapping is to be applied to a required portion of the human body, much skill is needed for such strapping treatment. If the strapping fails to be provided properly (for example, the application of the strapping to an improper portion, or unskilful and inadequate strapping), not only the intended purpose (i.e., the prevention of an injury and a remedy) is not achieved, but also this adversely affects various kinds of exercise, which may result in the interruption in blood circulation and nerval trouble, and besides this tends to adversely affect the muscle. Therefore, it is necessary that the strapping should be applied by those who have acquired the technique of strapping, and unskilled persons cannot easily apply the strapping.

When a supporter is to be used, a suitable supporter of a configuration corresponding to a required portion of the human body is chosen, and it can be quite easily attached to the required portion. However, although the supporter can provide a support in a direction perpendicular to the human body surface, it can not provide a required support force in a direction inclined relative to the human body surface, or in a spiral direction, or in a direction along the human body surface, so that sufficient support cannot be achieved because of insufficient tightening force. That is, such a supporter merely applies pressure to the human body, but cannot function to limit the movement of the relevant portion of the human body. Particularly when force is applied to the abdominal muscle or the like, the muscle is expanded and contracted, and therefore the muscle is expanded to increase its diameter. Therefore, when force is applied to the human body surface in perpendicular relation thereto, the expansion and contraction of the muscle are prevented, and fatigue is not alleviated, and in contrast this causes muscle fatigue.

In addition to the above-mentioned drawbacks of the prior art methods, another disadvantage has been encountered with the above strapping and the above supporters. More specifically, that portion of the human body surface to which the strapping or the supporter is applied is subjected to a tightening force, and therefore the difference between said that surface and the other surface portions appears because of the pressure difference, so that an unnatural step and profile can be recognized from the external appearance. This is quite unsightly, and particularly in the case of women, its silhouette cannot be kept beautiful.

WO-A-91/01794 is a document in the Japanese language of which the English-language abstract indicates that it discloses a covering article [for the human body] the main part of which is formed of a combination of elastic and non-elastic materials or of an elastic material only, in which specified parts of the elastic material are impregnated with treating agent so as to give different elasticity from the non-impregnated parts. The drawings include views of such an article applied to the knee, the ankle, the elbow, the wrist, and the shoulder.

US-A-4,910,802 discloses an exercise suit comprising a suit of unitary construction to envelop a human figure from the neck to the feet. The suit includes a plurality of elastic bands associated with each human appendage positioned on opposed sides of the arm portions of the suit and overlying a forward, side and back portion of each leg portion of the suit. A medially positioned elastic band secures the user's waist and there is an additional band about the user's chest. The various bands are positioned within enclosed conduits. The conduits for each band include a spaced region for enabling an adjustment buckle to adjust tension for each band.

The present invention provides a wearing article for wearing solely on the lower half of the human body in pressed relation to the body to provide a strapping function, the article including:
a tubular leg covering portion including a respective hard-to-stretch portion (1) extending longitudinally along each side thereof, and an easy-to-stretch portion (8) forming the remainder of the leg covering portion, the hard-to-stretch portions being joined together at part of the leg covering portion so as to cover that portion of the leg which is located above and adjacent to the knee; characterized in that:
the article covers the abdomen and the upper part of each leg including the thigh, a respective leg covering portion being provided for the upper part of each leg;
for each leg, the hard-to-stretch portion (1) extends longitudinally of the leg covering portion from an upper portion of the upper leg to a lower portion of the latter along the sides of the thigh, and
the parts of the hard-to-stretch portion (1) running along one and the other sides of the thigh leave the front of the thigh and the abdomen free of the hard-to-stretch material.

The hard-to-stretch portion is pressed against that portion (e.g. muscle or articulation) of the human body requiring a strapping treatment, in such a manner that the hard-to-stretch portion is extended along the muscle fibers over the region from the tendon to the central portion of the muscle. The other portions not requiring such a strapping treatment are covered by the easy-to-stretch portion.

With this construction, the wearing article, when worn on the human body, has the portion applying a high tightening force to the body surface, and the portion applying a low tightening force to the body surface. The former portion applying the high tightening force achieves a localized tightening effect similar to that achieved with a strapping treatment, thereby enabling the prevention and remedy of an injury. The wearing article can be provided in the form of tights for the lower half of the human body, a sock and overall tights. Therefore, upon wearing of this wearing article, even those who are not skilful in the technique of strapping can obtain an effect similar to that of a strapping treatment. The other portion of the integral wearing article except for the strapping portion is made of a two-way stretchable material which can stretch longitudinally and transversely, and therefore the strapping portion can not be recognized from an external view, and the wearing article can be smoothly worn on the body with a beautiful silhouette.

Ways of carrying out the invention will now be described, by way of example only, with reference to the single figure of the accompanying drawing, which figure is a front-elevational view of a long girdle according to the invention in which a hard-to-stretch cloth piece is provided so as to be disposed on the thighs and the lower leg portions.

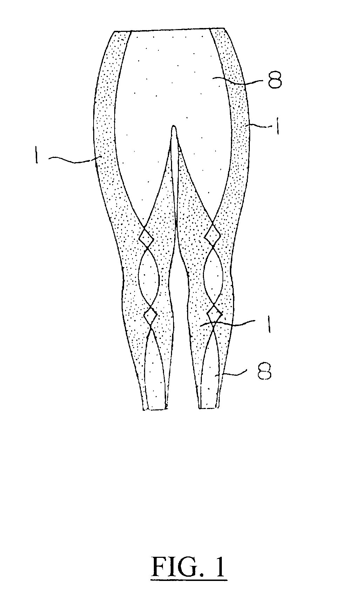

Referring to the drawing, Fig. 1 shows a wearing article with a strapping function for wearing on the lower half of the human body in pressed relation thereto. The article of figure 1 is a long girdle in which a hard-to-stretch cloth piece for providing a strapping function is provided so as to cover all of the front side of the lower half of the human body except for the front sides of the thighs, the knees and the front sides of the lower leg portions.

The long girdle has a tubular upper leg covering portion including a hard-to-stretch portion (1) forming a part of the upper leg covering portion, and an easy-to-stretch portion (8) which forms the remainder of the upper leg covering portion.

The hard-to-stretch portion (1) extends longitudinally of the upper leg covering portion along lateral portions thereof from an upper portion of the upper leg to a lower portion of the latter by way of lateral portions of the thigh.

The parts of the hard-to-stretch portion (1) which extend along one and the other of said lateral portions are joined together at a lower part of the upper leg covering portion so as to cover that portion of the upper leg which is located above and adjacent to the knee.

A tubular lower leg covering portion of the article has an upper end integral with the lower end of the upper leg covering portion. The lower leg covering portion includes a hard-to-stretch portion (1) forming a part of the lower leg covering portion, and an easy-to-stretch portion (8) forming the remainder of the lower leg covering portion. The hard-to-stretch portion (1) covers that portion of the lower leg which is located below and adjacent to the knee but does not cover the knee, and extends substantially longitudinally along the lower leg covering portion along lateral portions thereof but does not cover the front of the lower leg.

The hard-to-stretch portion (1) covers, in use, all of the front side of the lower half of the human body except for the front sides of the thighs, the knees and the front sides of the lower leg portions. As the drawing shows, the hard-to-stretch portion (1) does not cover the abdomen.

A construction is possible in which the wearing article is lined with the hard-to-stretch portion (1).

In this wearing article, the hard-to-stretch cloth piece is adapted to be provided not perpendicularly to the surface of the human body, but linearly, slantingly or spirally (or in other suitable form) generally along the muscle fibers, so that the hard-to-stretch cloth piece can be pressed against the relevant portion of the human body surface.

By way of background information, it may be noted that each of the supporters A shown in Figs. 7 to 10 of our European patent application No. EP-A-0 519 135 has a tubular shape, and is made of elastic (stretchable) materials (e.g. spandex) sewn together. The supporter A fits on the lower leg to extend between the knee and the ankle so as to tighten this portion. The predetermined portion of the supporter A is lined with the hard-to-stretch cloth piece 1 in the form of a band. With this arrangement, when the supporter A is worn on the lower leg, the hard-to-stretch portion (where the hard-to-stretch cloth piece 1 is provided) is pressed against the human body surface with a higher tightening force so as to provide a strapping function, whereas the other portion of the supporter A (that is, an easy-to-stretch cloth 8) is pressed against the human body surface with a lower tightening force. Thus, the supporter A has the two portions having different stretching (elastic) characteristics, respectively.

The said hard-to-stretch cloth piece 1 in European patent application No. EP-A-0 519 135 is in the form of a band (360 denier x 210 denier) having a generally uniform width, for example, of 4 to 6 cm throughout its length, and is made of a hard-to-stretch material, such as a spandex, which can be stretched longitudinally with a relatively high force. The supporter A is lined with the band-like hard-to-stretch cloth piece 1 by sewing, so that this cloth piece 1 is integrally connected to the supporter A.

In the supporters A of European patent application No. EP-A-0 519 135, the hard-to-stretch cloth piece 1 attached to the inner side thereof provides a higher tightening force than the other portion (that is, the easy-to-stretch cloth 8) of the supporter A, and therefore the hard-to-stretch cloth piece 1 can provide strong stretching characteristics almost the same as achieved with a strapping treatment. When the band-like hard-to-stretch cloth piece 1 has a very much stronger stretching force than the easy-to-stretch cloth 8, a strapping treatment for achieving a stronger support effect can be provided. More specifically, the hard-to-stretch cloth piece 1 is stretched or expanded, and then in this condition this cloth piece 1 is sewn to the easy-to-stretch cloth 8. With this arrangement, when the supporter A is worn on the lower leg, the hard-to-stretch cloth piece 1 is stretched, and therefore the hard-to-stretch cloth piece 1 tends to contract, so that a strong contracting force is applied in the direction of the muscle fibers.

As described above, in the supporters A, band-like hard-to-stretch cloth piece 1, having uniform width throughout its length, is spirally provided. In other wearing articles to be worn on a required portion of the human body in pressed relation to the human body surface, the hard-to-stretch cloth piece 1 of a required shape is adapted to be provided on a required articulation or muscle so as to extend generally in the direction of the muscle fibers from the tendon to the central portion of the muscle. By doing so, a strapping function can be achieved. The hard-to-stretch cloth piece 1 is applied to the human body surface along the muscle fibers, and is applied under pressure to part or the whole of the muscle fibers. The hard-to-stretch cloth piece 1 need only to be extended longitudinally in the direction of the muscle fibers. When the hard-to-stretch cloth piece 1 is provided perpendicular to the human body surface, the muscle fatigue cannot be alleviated, and also a remedy for an injury can not be achieved. In other words, the dissipation of energy and the accumulation of a fatigue substance (e.g. lactic acid) cause muscle fatigue. The recovery from the fatigue can be promoted by promoting the flow of the blood and the lymph to rapidly remove the fatigue substance. When the skin is subjected to a massage effect by applying a proper degree of pressure thereto, the flow of the blood and the lymph can be promoted, and the recovery from the fatigue can be promoted. However, when the muscular strength is exerted, the muscle fibers are contracted, and the muscle bundle becomes thicker, and it is not desirable to hold the central portion of the muscle (i.e., the thickened muscle bundle).

In Figures 7 to 10 of European patent application No. EP-A-0 519 135, although the hard-to-stretch cloth piece 1 is integrally sewn to the supporter A by lining, other possibilities exist. For example, there can be adopted a mold knitting method by which a high-power portion and a low-power portion are provided in a supporter, a molding method by which a high-power portion and a lower-power portion is provided in a supporter while providing a three-dimensional configuration, a method in which the hard-to-stretch cloth piece 1 is sewn to the outer side of the supporter, and a method in which instead of the hard-to-stretch cloth piece 1, a coating of a synthetic resin is applied to the supporter to prevent the stretching.

Further, when wearing the wearing article of the present invention, the same effect as the strapping treatment can be provided at the required portion extending along the central portion of the muscle, and besides upon wearing of the wearing article, even those who are not skilful in the strapping treatment can have the strapping support effect, and also tightening pressure can be applied to part of the body.

Further, since the band-like hard-to-stretch cloth piece 1 which is strong and can apply a high tightening force is used, the motion of the muscle is suitably supported and controlled. Also, that portion except for the hard-to-stretch cloth piece 1 which portion covers and fits on the body surface is as elastic (stretchable) as that obtained with the prior art, and therefore excellent fitting, heat-retaining and protective properties are obtained.

Further, the one-piece cloth, having the high-power portion and the lower-power portion continuous with each other, is used, and therefore the silhouette upon wearing is smooth and beautiful, and no profile of the high-power portion or the low-power portion appears on the outer surface of the wearing article.

## Claims

1. A wearing article for wearing solely on the lower half of the human body in pressed relation to the body to provide a strapping function, the article including:
a tubular leg covering portion including a respective hard-to-stretch portion (1) extending longitudinally along each side thereof, and an easy-to-stretch portion (8) forming the remainder of the leg covering portion, the hard-to-stretch portions being joined together at part of the leg covering portion so as to cover that portion of the leg which is located above and adjacent to the knee; **characterized in that**:
the article covers the abdomen and the upper part of each leg including the thigh, a respective leg covering portion being provided for the upper part of each leg;
for each leg, the hard-to-stretch portion (1) extends longitudinally of the leg covering portion from an upper portion of the upper leg to a lower portion of the latter along the sides of the thigh, and
the parts of the hard-to-stretch portion (1) running along one and the other sides of the thigh leave the front of the thigh and the abdomen free of the hard-to-stretch material.

2. A wearing article according to claim 1, further including, for each leg, a respective tubular lower leg covering portion having an upper end integral with the lower end of the associated upper leg covering portion, the lower leg covering portion including a hard-to-stretch portion (1) forming a part of the lower leg covering portion, and an easy-to-stretch portion (8) forming the remainder of the lower leg covering portion, the heavily-stretchable portion (1) covering that portion of the lower leg which is located below and adjacent to the knee but not covering the knee, and extending substantially longitudinally along the lower leg covering portion along lateral portions thereof but not covering the front of the lower leg.

3. A wearing article as claimed in claim 1, in which the article is a long girdle in which the hard-to-stretch portion (1) covers, in use, all of the front side of the lower half of the human body except for the front sides of the thighs, the abdomen, the knees and the front sides of the lower leg portions.

4. A wearing article according to any preceding claim, in which said hard-to-stretch portion (1) is applied as a lining to said wearing article.

## Patentansprüche

1. Kleidungsartikel, der lediglich an der unteren Hälfte des menschlichen Körpers in einer an dem Körper angedrückten Beziehung getragen wird, um eine Bandagefunktion vorzusehen, wobei der Artikel folgendes hat:
einen röhrenartigen das Bein bedeckenden Abschnitt, der einen jeweiligen kaum dehnbaren Abschnitt (1), der sich in Längsrichtung entlang jeder Seite von diesem erstreckt, und einen leicht dehnbaren Abschnitt (8) hat, der den Rest des das Bein abdeckenden Abschnittes bildet, wobei die kaum dehnbaren Abschnitte miteinander an einem Teil des das Bein abdeckenden Abschnittes so verbunden sind, dass jener Abschnitt des Beins bedeckt ist, der sich oberhalb und benachbart zu dem Knie befindet,
**dadurch gekennzeichnet, dass**
der Artikel den Bauch und den oberen Teil von jedem Bein inklusive des Schenkels abdeckt, wobei ein jeweiliger das Bein abdeckender Abschnitt für den oberen Teil von jedem Bein vorgesehen ist;
für jedes Bein sich der kaum dehnbare Abschnitt (1) in der Längsrichtung des das Bein abdeckenden Abschnittes von einem oberen Abschnitt des Oberschenkels zu einem unteren Abschnitt des letztgenannten entlang der Seiten des Schenkels erstreckt, und
die Teile des kaum dehnbaren Abschnittes (1), die entlang der einen oder der anderen Seite des Schenkels verlaufen, die Vorderseite des Schenkels und des Bauchs von dem kaum dehnbaren Material frei lassen.

2. Kleidungsartikel gemäß Anspruch 1, der des weiteren für jedes Bein einen jeweiligen röhrenartigen den Unterschenkel abdeckenden Abschnitt mit einem oberen Ende hat, das mit dem unteren Ende des zugehörigen den Oberschenkel abdeckenden Abschnittes einstückig ist, wobei der den Unterschenkel abdeckende Abschnitt einen kaum dehnbaren Abschnitt (1), der ein Teil des den Unterschenkel abdeckenden Abschnittes bildet, und einen leicht dehnbaren Abschnitt (8) hat, der den Rest des den Unterschenkel abdeckenden Abschnittes bildet, wobei der schwer dehnbare Abschnitt (1) den Abschnitt des Unterschenkels abdeckt, der sich unterhalb und benachbart zu dem Knie befindet, jedoch das Knie nicht abdeckt, und sich im wesentlichen in der Längsrichtung entlang des den Unterschenkel abdeckenden Abschnittes entlang seiner Seitenabschnitte erstreckt aber nicht die Vorderseite des Unterschenkels abdeckt.

3. Kleidungsartikel gemäß Anspruch 1, wobei
der Artikel eine lange Korseletthose ist, bei dem der kaum dehnbare Abschnitt (1) bei der Anwendung die gesamte Vorderseite der unteren Hälfte des menschlichen Körpers mit Ausnahme der Vorderseiten der Schenkel, des Bauchs, der Knie und der Vorderseiten der Unterschenkelabschnitte abdeckt.

4. Kleidungsartikel gemäß einem der vorherigen Ansprüche, bei dem der kaum dehnbare Abschnitt (1) als eine Fütterung an dem Kleidungsartikel angebracht ist.

## Revendications

1. Un article vestimentaire à porter uniquement sur la moitié inférieure du corps humain, en relation pressée envers le corps, afin de fournir une fonction de contention, l'article comprenant:
un élément de couverture de jambe, tubulaire, incluant un élément difficile à étirer (1) respectif, s'étendant longitudinalement sur chaque côté de celui-ci, et un élément aisé à étirer (8) formant le reste de l'élément de couverture de jambe, les éléments difficiles à étirer étant reliés ensemble en une partie d'un élément de couverture de jambe, de manière à couvrir la partie de la jambe située au-dessus et près du genou; **caractérisé en ce que** :
l'article couvre l'abdomen et la partie supérieure de chaque jambe y compris la cuisse, un élément de couverture de jambe respectif étant prévu pour la partie supérieure de chaque jambe;
pour chaque jambe, l'élément difficile à étirer (1) s'étend dans la direction longitudinale d'un élément de couverture de jambe, depuis une partie supérieure de la partie supérieure de la jambe jusqu'à une partie inférieure de cette dernière, sur les côtés de la cuisse, et
les parties de l'élément difficile à étirer (1) s'étendant sur l'un et l'autre côté de la cuisse, laissant la cuisse et l'abdomen exempts de tout matériau difficile à étirer.

2. Un article vestimentaire selon la revendication 1, comprenant en outre, pour chaque jambe, un élément de couverture de jambe inférieur tubulaire respectif, ayant une extrémité supérieure réalisée d'une seule pièce avec l'extrémité inférieure de l'élément de couverture de jambe supérieur associé, l'élément de couverture de jambe inférieure comprenant une partie difficile à étirer (1) formant une partie de l'élément de couverture de jambe inférieur, et un élément aisé à étirer (8) formant le reste de l'élément de couverture de jambe inférieur, l'élément difficile à étirer (1) couvrant la partie de la jambe inférieure située au-dessous et au voisinage du genou, mais ne couvrant pas le genou et s'étendant sensiblement dans la direction longitudinale, le long de l'élément de couverture de jambe inférieure, sur ses parties latérales, mais ne couvrant pas l'avant de la jambe inférieure.

3. Un article vestimentaire selon la revendication 1, dans lequel l'article est une gaine allongée, dans lequel la partie difficile à étirer (1) couvre, en utilisation, la totalité de la face avant de la moitié inférieure du corps humain, sauf pour ce qu'il concerne les faces avant des cuisses, l'abdomen, les genoux et les faces avant des parties de jambe inférieures.

4. Un article vestimentaire selon l'une quelconque des revendications précédentes, dans lequel l'élément difficile à étirer (1) est appliqué sous forme de revêtement audit article vestimentaire.
